# DEMANDE DE BREVET EUROPEEN

(11) **EP 1 304 100 A1**
(43) Date de publication de la demande: **23.04.2003**
(21) Numéro de dépôt: 02292444.3
(22) Date de dépôt: 04.10.2002
(51) Int. Cl.: A61K 7/06

(54) **Traitement du cheveu par ajout d'enzymes exogènes, d'alcools et d'acides ou de triglycérides**

(30) Priorité: 11.10.2001 FR 0113112; 11.10.2001 FR 0113113
(71) Demandeur: L'OREAL, 75008 Paris (FR)
(72) Inventeur: Kaba, Geneviève, 94200 Ivry s/ Seine (FR); Semeiria, Didier, 93190 Livry Gargan (FR); Duvault, Yolanda, 93600 Aulnay Sous Bois (FR)
(74) Mandataire: Casalonga, Axel

(57) **Abrégé**

La présente invention concerne des compositions de traitement des matières kératiniques par ajout d'au moins une enzyme exogène, d'alcools à longues chaînes, et d'acides à longues chaînes ou de triglycérides qui donne lieu à une réaction enzymatique directement sur le cheveu, ainsi qu'à des procédés de traitement des cheveux mettant en oeuvre ces compositions.

## Description

La présente invention concerne des compositions de traitement des matières kératiniques par ajout d'au moins une enzyme exogène, d'alcools à longues chaînes, et d'acides à longues chaînes ou de triglycérides qui donne lieu à une réaction enzymatique directement sur le cheveu, ainsi qu'à des procédés de traitement des cheveux mettant en oeuvre ces compositions.

Les cheveux sont soumis à des agressions continuelles qui peuvent les rendre cassants, difficiles à démêler ou ternes. C'est pourquoi il est important de trouver des produits capables de conditionner la chevelure en la laissant facile à coiffer, souple, douce au toucher et lustrée. Il serait également intéressant que les propriétés de douceur et brillance de ces produits perdurent pendant plusieurs shampooings.

On a constaté que l'aspect brillant et doux de la fibre traitée est particulièrement important si l'on apporte sur le cheveu des esters à longues chaînes.

On a déjà proposé d'utiliser des enzymes comme agents de conditionnement pour la chevelure. De telles compositions sont décrites notamment dans la demande de brevet WO 00-64 405 où la composition qui améliore l'aspect du cheveu nécessite la présence d'une enzyme endogène présente dans la fibre du cheveu.

La demande de brevet DE 1 982 474 décrit l'utilisation d'enzymes exogènes afin d'inverser le processus d'hydrolyse du sébum par le biais d'une réestérification du glycérol et des acides gras du sébum générés *in situ.*

D'autres compositions de traitement des cheveux dont le procédé fait intervenir une réaction enzymatique ont été décrites, notamment dans les demandes de brevets EP 293 0 01, JP 081 173 787, et EP 391 4 31.

La demanderesse a découvert que la formation *in situ* d'esters gras en mettant en association des acides à longues chaînes ou des triglycérides, des alcools à longues chaînes et au moins une ou plusieurs enzymes exogènes susceptibles de produire une réaction d'estérification, améliore l'aspect brillant et doux de la fibre capillaire ainsi traitée, la rend facile à coiffer et lui apporte de la discipline.

La demanderesse a découvert de plus, de façon surprenante, que lorsque la réaction d'estérification ou de transestérification est faite *in situ* directement sur le cheveu, le résultat sur les matières kératiniques en terme de brillance et de douceur est supérieur à un simple dépôt des esters correspondants synthétisés à part. D'autre part, ces propriétés sont conservées après plusieurs shampooings.

L'invention a encore pour objet un procédé de traitement des cheveux, mettant en oeuvre ces compositions.

D'autres objets de la présente demande ressortiront à la lecture de la description et des exemples qui suivent.

Les compositions cosmétiques selon l'invention contiennent au moins un alcool de formule R₁-OH, où R₁ est un groupement alkyle en C₃ à C₃₂, linéaire ou ramifié, saturé ou non saturé, polyoxyéthylénés ou non, éventuellement substitué, au moins un acide de formule générale R₂-COOH, où R₂ est un groupement alkyle en C₃ à C₃₁, linéaire ou ramifié, saturé ou non saturé, éventuellement substitué, avec un nombre d'atomes de carbone de (R₁ + R₂) ≥ 8, ou au moins un triglycéride, et au moins une enzyme exogène susceptible de provoquer une réaction d'estérification.

Les compositions cosmétiques selon l'invention contiennent au moins un alcool de formule R₁-OH, où R₁ est un groupement alkyle en C₃ à C₃₂, linéaire ou ramifié, saturé ou non saturé, polyoxyéthylénés ou non, éventuellement substitué, au moins un acide de formule générale R₂-COOH, où R₂ est un groupement alkyle en C₃ à C₃₁, linéaire ou ramifié, saturé ou non saturé, éventuellement substitué, avec un nombre d'atomes de carbone de (R₁ + R₂) ≥ 8, et au moins une enzyme susceptible de provoquer une réaction d'estérification.

Les compositions cosmétiques selon l'invention contiennent au moins un alcool de formule R₁-OH, où R₁ est un groupement alkyle en C₃ à C₃₂, linéaire ou ramifié, saturé ou non saturé, polyoxéthyléné ou non, éventuellement substitué, au moins un triglycéride, et au moins une enzyme exogène susceptible de provoquer une réaction d'estérification.

Les alcools utilisables selon l'invention sont choisis notamment parmi les alcools suivants : hexanol, ethyl-2hexanol, octanol, butyl-2 octanol, nonanol, décanol, hexyl-2 décanol, undécanol, dodécanol, octyl-2 dodécanol, tridécanol, tétradécanol, décyl-2 tétradécanol, pentadécanol, hexadécanol, dodécyl-2 hexadécanol, octadécanol, tétradecyl-2 octadécanol, nonadécanol, tricosanol, hexacosanol, triacontanol, alcools arachinylique, béhénylique, lignocérylique, palmitoléïlique, oléïlique, élaidylique, ricinoléylique, linoléylique, linolénylique, arachidonylique, érucylique, les alcools polyoxyéthylénés tels que le polyoxyéthylène lauryl ether, le polyoxyéthylène cétyl éther, le polyoxyéthylène stéaryl éther, les dérivés vendus sous la dénomination "Brij" par la société ICI.

Les acides utilisables selon l'invention sont choisis notamment parmi les acides suivants : acides hexanoique, heptanoique, octanoique, pélargonique, décanoique, undecanoique, dodécanoique, tétradécanoique, hexadécanoique, octadécanoique, eicosanoique, docosanoique, tétracosanoique, hexacosanoique, triacontanoique, undécénoique, palmitique, palmitoléique, oléique, élaidique, linoléique, linolénique, ricinoléique, arachidonique, érucique, brassidique, nervonique, stéarique, 12-hydroxystéarique, isostéarique, 2-butyloctanoique, 2-hexyldécanoique, 2-décylmyristique, 2-laurylhexadécanoique, 2-tétraéecyloctadécanoique, 2-pentadécylnonadécanoique, 2-hexadécyleicosanoique, octyl-2-dodécanoique.

Les triglycérides utilisables selon l'invention sont choisis notamment parmi les triglycérides suivants : trihexanoate de glycérol, tricaprylate de glycérol, trinonanoate de glycérol, tridécanoate de glycérol, triundécanoate de glycérol, trilaurate de glycérol, tripendadécanoate de glycérol, trimyristate de glycérol, tritridécanoate de glycérol, tripalmitate de glycérol, tristéarate de glycérol, triarachidonate de glycérol, tribéhénate de glycérol, tri(cis-9 tétradécénoate) de glycérol, tri(cis-9 hexadécénoate) de glycérol, tri(trans hexadécénoate) de glycérol, trioléate de glycérol, tri(trans-9 octadécénoate) de glycérol, tri(cis,cis-9-12 octadécanediénoate) de glycérol, tri(cis, cis, cis, cis-5, 8, 11, 14 eicosanetétraénoate) de glycérol tri(cis-13 docosénoate) de glycérol, tri(cis-15 tétracosénoate) de glycérol, triglycérides d'huiles de coprah, de palme, de soja, de jojoba, de colza, de palmiste, d'amande, de ricin, de tournesol, de carthame, de bourrache, de germe de maïs, trigycérides de suif, beurre de cacao, de karité.

Ces produits sont commercialisés notamment par la société Sigma.

Les enzymes susceptibles de provoquer l'estérification sont choisies notamment parmi les lipases, estérases, protéases. Elles peuvent être libres ou immobilisées sur divers supports.

Comme protéases selon l'invention, on peut citer entre autres, les protéases d'origine animale pepsine, chymotrypsine, trypsine, rennine, pancréatine, collagénase, élastase ...), d'origine végétale (papaine, chymopapaine, bromelaine, ficine, ...), ou d'origine microbienne (fongique, bactérienne, de levure, tels *Aspergillus, Penicillium, Rhizopus ou Mucor,* ...) ; Ces diverses protéases sont commercialisées par les sociétés Amano, Novo, Sigma, Boehringer, Nagase, Green cross corp,...

Comme lipases selon l'invention, on peut citer entre autres, les lipases d'origine animale (pancréatique, du lait, ...), d'origines végétale ou microbienne (fongique, bactérienne ou levure ) telles que les lipases fongiques *Mucor miehi, Aspergilus niger, Rhizopus arrhizus, Rhizopus delemar, Rhizopus japonicus, Rhizopus oryzae, Rhizopus sp., Geotrichum candidum,* ..., les lipases bactériennes *Chromobactérium viscosum, Arthrobacter ureafaciens, Achromobacter sp., Pseudomonas aeruginosa, Pseudomonas fluorescens, Pseudomonas sp., Alcaligenes sp.,* ..., les lipases de levures *Candida cylindracea, Candida rugosa, Candida lipolytica, Candida antarctica*...Ces diverses lipases sont commercialisées par les sociétés Amano, Novo, Sigma, Fluka, Biocatalysts, Godo Shusei, Meito sangyo, ...

Comme estérases selon l'invention, on peut citer entre autres, les estérases de foie de porc, de foie de lapin, de pancréas, commercialisées par la société Sigma.

Un grand nombre d'enzymes industrielles, utiles à l'invention, sont répertoriées dans l'ouvrage « Industrial enzymes and their applications « de Helmuth UHLIG en 1998 chez John WILEY Inc. New York. Des enzymes peuvent éventuellement être issues également de recombinaisons génétiques (enzymes recombinantes).

Lorsque ces enzymes sont utilisées sous forme immobilisée sur support, un très grand nombre de supports sont possibles tels que, entre autres, des résines, de la terre de diatomée de la marque Célite, du Nylon, du verre poreux, de la cellulose, du polyethyleneglycol...) et répertoriés dans la revue « Journal of American Oil Chemist Society » 1990 Vol. 67 N° 12 p.890-910 ; parmi celles-ci, on peut retenir les Lipozymes RM IM, IM 60, et la Novozym 435 de chez NOVO Nordisk ;

La quantité d'enzymes présente dans la composition cosmétique selon l'invention, est comprise entre 0,0001 et 50 % en poids par rapport au poids du mélange en poids d'acides et d'alcools ou de triglycérides et d'alcools. De préférence, la quantité d'enzymes est comprise entre 0,1 et 20 % en poids par rapport au poids du mélange en poids d'acides et d'alcools ou de triglycérides et d'alcools.

Dans la composition cosmétique selon l'invention, la proportion d'acides peut varier entre 1 et 99 % par rapport au mélange en poids d'acides et d'alcools. De préférence, la proportion d'acides peut varier entre 30 et 70 % par rapport au mélange en poids d'acides et d'alcools.

Dans la composition cosmétique selon l'invention, la proportion de triglycérides peut varier entre 1 et 99 % par rapport au mélange en poids d'alcools et de triglycérides. De préférence, la proportion de triglycérides peut varier entre 30 et 70 % par rapport au mélange en poids d'alcools et de triglycérides.

Dans la composition cosmétique selon l'invention, la proportion d'alcools peut varier entre 1 et 99 % par rapport au mélange en poids du mélange en poids d'acides et d'alcools ou de triglycérides et d'alcools.

De préférence, la proportion d'alcools peut varier entre 30 et 70 % par rapport au mélange en poids du mélange en poids d'acides et d'alcools ou de triglycérides et d'alcools.

Outre les constituants obligatoires précités, la composition selon l'invention peut comprendre des adjuvants choisis parmi les huiles carbonées, siliconées, fluorées, les gélifiants, les épaississants, les émollients, les adoucissants, les antioxydants, les opacifiants, les stabilisants, les polymères ioniques, non-ioniques ou amphotères, les vitamines, les parfums, les conservateurs, les colorants, les pigments ou tout autre adjuvant habituellement utilisé en cosmétique, compatibles avec le ou les enzymes utilisés.

La composition cosmétique selon l'invention se présente sous forme de lotions, de gels ou de crèmes à rincer ou non.

Un autre objet de l'invention est un dispositif à plusieurs compartiments (au moins deux) contenant chacun un ou deux des composants suivants :
- un ou des alcools de formule R1-OH où R₁ est un groupement alkyle de C₃ à C₃₂, linéaire ou ramifié, saturé ou non-saturé, polyoxyéthyléné ou non, éventuellement substitué.
- un ou des acides de formule générale R₂-COOH où R₂ est un groupement alkyle de C₃ à C₃₁, linéaire ou ramifié, saturé ou non-saturé, éventuellement substitué avec le nombre de carbones de (R₁ + R₁) ≥ 8 ou un ou des triglycérides
- une ou des enzymes exogènes susceptibles de provoquer l'estérification, l'ensemble du dispositif renfermant au moins un acide, un alcool ou un triglycéride et une enzyme. Le mélange entre les différents composants du dispositif est effectué juste avant emploi.

Un autre objet de l'invention est l'utilisation des compositions selon l'invention, pour apporter à la fibre capillaire un aspect doux et brillant et facile à coiffer.

Un autre objet de l'invention est un procédé de traitement cosmétique des cheveux pour apporter un aspect brillant et doux à la fibre. Le mode d'utilisation des compositions cosmétiques selon l'invention est le suivant : on mélange les acides, les alcools ou les triglycérides et les enzymes juste avant l'utilisation (entre 1 seconde et 5 minutes), et on applique immédiatement la composition résultante sur les fibres kératiniques, on laisse agir pendant 1 à 60 minutes à une température comprise entre 20 et 65°C, de préférence entre 30 et 65°C, et on procède éventuellement au rinçage à l'eau.

Un autre objet de l'invention est que le procédé de traitement fait intervenir une réaction d'estérification ou de transestérification qui se produit *in situ* sur le cheveu.

Les exemples suivants sont destinés à illustrer l'invention, sans toutefois présenter un caractère limitatif.

### Exemple 1 : Rémanence mesurée pour une réaction acides/alcools/enzymes effectuée in situ sur le cheveu

Les mèches de cheveu utilisées sont choisies pour la qualité du sébum chromatographié.

Dans un flacon de 200ml à large ouverture, on mélange
- 380 mg d'alcool gras linéaire en C12
- 380 mg d'alcool gras linéaire en C14
- 190 mg d'acide gras linéaires saturés en C12
- 190 mg d'acide gras linéaires saturés en C14
- 190 mg d'acide gras linéaires saturés en C16
- 230 mg d'acide gras linéaires mono-insaturés en C18
- 150 mg d'acide gras linéaires mono-insaturés en C22
le mélange est chauffé à 50°C jusqu'à l'obtention d'une solution homogène puis 0,5 mL d'eau est ajouté et 120 mg d'enzyme immobilisée lipozyme RM IM. L'ensemble est vigoureusement agité 2 fois 2 minutes et déposé sur une mèche de 0,5 g de cheveu enroulée au fond du flacon et ce dernier est porté à 38°C pendant 30 minutes. La mèche est ensuite retirée et abondamment rincée sous le robinet à 40°C. Elle est ensuite lavée en deux temps avec du shampooing Dop à 3% (2 mL à deux reprises). Rincée, elle est séchée sous sèche-cheveux.

5 portions de 1 cm de 5 cheveux différents sont coupés au milieu du cheveu préalablement mesuré et introduits dans l'insert d'un chromatographe en phase gazeuse Hewlet-Packard 5890 série II.

| Nombre de shampooings après traitement | Rémanence au shampooing de la marque DOP à 3% (exprimée en µg de cires par g de cheveu) |
|---|---|
| 1 | 500 |
| 3 | 400 |
| 5 | 250 |
| 7 | 200 |

Cette expérience montre qu'au bout de 5 shampooings, au moins 50 % des cires déposées sont encore fixées sur le cheveu. Il y a en fait rémanence des produits jusqu'à 7 shampooings.

### Exemple 2 : Rémanence mesurée pour une réaction triglycérides/alcoosl/enzymes effectuée in situ sur le cheveu :

Dans un flacon de 200 mL à large ouverture, on mélange
- 0,4 g d'huile de colza
- 0,4 g d'huile de coprah
- 0,4 g d'huile de palme
- 1 g de dodécanol
- 1 g de tétradécanol

Le mélange est ensuite chauffé à 50°C jusqu'à obtention d'une solution homogène, puis 1,2 mL d'eau est ajouté ainsi que 300 mg d'enzyme immobilisée RM IM.

L'ensemble est vigoureusement agité deux fois deux minutes, puis déposé sur une mèche de 1,3 g de cheveu enroulée au fond du flacon. Ce dernier est porté à 38°C pendant 30 minutes. La mèche est ensuite retirée et abondamment rincée sous le robinet à 40°C. Elle est lavée ensuite deux fois avec du shampoing de la marque Dop à 3% (2mL à deux reprises). Rincée, elle est séchée sous sèche-cheveux.

5 portions de 1 cm de 5 cheveux différents sont coupés au milieu du cheveu préalablement mesuré et introduits dans l'insert d'un chromatographe en phase gazeuse Hewlet-Packard 5890 série II.

| Nombre de shampooings après traitement | Rémanence au shampooing de la marque DOP à 3% (exprimée en µg d'esters d'acides gras par g de cheveu) |
|---|---|
| 1 | 3000 |
| 3 | 300 |
| 5 | 200 |
| 7 | 200 |

Il y a rémanence des produits jusqu'à 7 shampooings.

### Exemple 3 : Rémanence mesurée si la solution acides/alcools/enzymes est préparée au préalable

Le but de cette expérience est de vérifier si la solution acides/alcools/enzymes est aussi efficace au niveau de la rémanence sur le cheveu si elle a été préparée à l'avance au lieu d'être ex-temporanée.

On mélange dans un bêcher :
- 760 mg d'alcool en C12
- 760 mg d'alcool en C14
- 380 mg d'acide gras en C12
- 380 mg d'acide gras en C14
- 380 mg d'acide gras en C16
- 500 mg d'acide gras mono-insaturé en C18
- 50 mg d'acide gras mono-insaturé en C22
- 1 mL d'eau
- et 240 mg de lipozyme RMIM

Le mélange obtenu est placé dans une étuve à 38°C pendant 30 minutes.

L'étude de la rémanence aux shampooings est effectuée de la même manière que dans l'expérience 1.

L'image chromatographique de la lotion obtenue en bécher montre que les cires sont formées avant leur dépôt sur le cheveu. De plus, après un shampooing Dop à 3%, moins de 100 µg de cires sont rémanentes sur la chevelure. Et après 5 lavages, il n'y a plus de cires rémanentes détectables.

### Exemple 4 : Rémanence mesurée si la solution triglycérides/alcoosl/enzymes est préparée au préalable

Le but de cette expérience est de vérifier si la solution triglycérides/alcoosl/enzymes est aussi efficace au niveau de la rémanence sur le cheveu si elle a été préparée à l'avance au lieu d'être ex-temporanée.

Dans un flacon de 200 mL à large ouverture, on mélange
- 0,4 g d'huile de colza
- 0,4 g d'huile de coprah
- 0,4 g d'huile de palme
- 1 g de dodécanol
- 1 g de tétradécanol

Le mélange est ensuite chauffé à 50°C jusqu'à obtention d'une solution homogène, puis 1,2 mL d'eau est ajouté ainsi que 300 mg d'enzyme immobilisée RM IM.

L'ensemble est vigoureusement agité deux fois deux minutes, puis placé à 38°C durant 30 minutes dans une étuve.

L'image chromatographique de la lotion obtenue dans le flacon montre que les cires sont formées avant leur dépôt sur le cheveu.

L'étude de la rémanence aux shampooings est effectuée de la même manière que dans l'expérience 1.

Après 1 shampooing, il y a moins de 100 µg d'esters d'acides gras par gramme de cheveu. Après 5 shampooings, les esters d'acides gras rémanents sont en limite de détection.

### Exemple 5 : Evaluation de l'action de lipozyme RM IM sur le dépôt de cires sur le cheveu

La même expérience que celle décrite dans l'exemple 1 a été faite en présence ou non d'enzyme immobilisée lipozyme RM IM dans la solution à déposer sur le cheveu.

Les profils de chromatographie montrent qu'en absence de lipozyme RM IM, il n'y a pas formation de cires sur le cheveu.

### Exemple 6 : Evaluation de l'action de lipozyme RM IM sur le dépôt d'esters d'acides gras sur le cheveu

La même expérience que celle décrite dans l'exemple 2 a été faite en présence ou non d'enzyme immobilisée lipozyme RM IM dans la solution à déposer sur le cheveu.

Les profils de chromatographie montrent qu'en absence de lipozyme RM IM, il n'y a pas formation d'esters d'acides gras sur le cheveu.

### Exemple 7 : Evaluation de l'action des acides gras endogènes sur le dépôt de cires sur le cheveu

La même expérience que celle décrite dans l'exemple 1 a été faite en présence ou non d'acides gras exogènes dans la solution à déposer sur le cheveu.

Les profils de chromatographie montrent qu'en absence d'acides gras exogènes, il n'y a pas formation de cires sur le cheveu. Ainsi, la formation de cires et leur rémanence sur le cheveu dépend de la présence dans la solution utilisée à la fois d'acide gras, d'alcools gras et d'enzymes exogènes.

### Exemple 8 : Evaluation des modifications cosmétiques en métrologie capillaire avec le mélange alcools gras, acides gras et enzymes

L'évaluation comparative des propriétés cosmétiques a été réalisée sur des mèches délipidées et non délipidées avec le mélange alcools gras, acides gras et enzyme par test de sonomètre. L'objectif de ce test est de quantifier le bruit du passage d'un peigne dans des cheveux démêlés. Ce bruit peut être plus ou moins fort selon l'état de surface du cheveu, de la qualité des pointes, de la matière déposée même en très faible quantité.

Un peigne est associé à un microphone. Le son capté par ce microphone lors du passage du peigne dans les cheveux est calibré par un sonomètre dont la sortie analogique est branchée sur un intégrateur de tension. Le résultat affiché correspond à la somme des différents bruits recueillis au cours de la mesure.

Moyennes et écarts types du bruit mesuré :

| **Formules** | **Moyenne** | **Ecart-type** |
|---|---|---|
| Délipidés Témoin | 1546 | 158 |
| Délipidés + traitement enzymatique + acides et alcools | 330 | 124 |
| | | |
| Non délipidés Témoins | 2101 | 242 |
| Non délipidés + traitement enzymatique + acides et alcools | 906 | 206 |

Il est clairement montré que le traitement acides + alcools et enzyme provoquent une diminution du bruit généré donc l'amélioration de l'état de surface des cheveux. Cet effet est obtenu aussi bien sur les cheveux naturels délipidés que sur les cheveux naturels non délipidés.

### Exemple 9 : Evaluation des modifications cosmétiques en métrologie capillaire avec le mélange alcools gras, triglycérides et enzymes

L'évaluation comparative des propriétés cosmétiques a été réalisée sur des mèches délipidées et non délipidées avec le mélange alcools gras, triglycérides et enzymes par test de sonomètre. L'objectif de ce test est de quantifier le bruit du passage d'un peigne dans des cheveux démêlés. Ce bruit peut être plus ou moins fort selon l'état de surface du cheveu, de la qualité des pointes, de la matière déposée même en très faible quantité.

Un peigne est associé à un microphone. Le son capté par ce microphone lors du passage du peigne dans les cheveux est calibré par un sonomètre dont la sortie analogique est branchée sur un intégrateur de tension. Le résultat affiché correspond à la somme des différents bruits recueillis au cours de la mesure.

Moyennes et écarts types du bruit mesuré :

| **Formules** | **Moyenne** | **Ecart-type** |
|---|---|---|
| Délipidés Témoin | 1546 | 158 |
| Délipidés + traitement enzymatique + huiles et alcools | 243 | 103 |
| | | |
| Non délipidés Témoins | 2101 | 242 |
| Non délipidés + traitement enzymatique + huiles et alcools | 1500 | 249 |

Il est clairement montré que le traitement huiles (triglycérides) + alcools et enzyme provoque une diminution du bruit généré donc l'amélioration de l'état de surface des cheveux. Il est important de noter que cet effet est obtenu aussi bien sur les cheveux naturels délipidés que sur les cheveux naturels non délipidés.

## Revendications

1. Composition cosmétique comprenant :
a) au moins un alcool de formule R₁-OH où R₁ est un groupement alkyle de C₃ à C₃₂, linéaire ou ramifié, saturé ou non-saturé, polyoxyéthyléné ou non, éventuellement substitué.
b) au moins un acide de formule générale R₂-COOH où R₂ est un groupement alkyle de C₃ à C₃₁, linéaire ou ramifié, saturé ou non-saturé, éventuellement substitué avec le nombre de carbones de (R₁ + R₁) ≥ 8 ou au moins un triglycéride
c) au moins une enzyme exogène susceptible de provoquer l'estérification.

2. Composition cosmétique selon la revendication 1, comprenant :
a) au moins un alcool de formule R₁-OH où R₁ est un groupement alkyle de C₃ à C₃₂, linéaire ou ramifié, saturé ou non-saturé, polyoxyéthyléné ou non, éventuellement substitué.
b) au moins un acide de formule générale R₂-COOH où R₂ est un groupement alkyle de C₃ à C₃₁, linéaire ou ramifié, saturé ou non-saturé, éventuellement substitué avec le nombre de carbones de (R₁ + R₁) ≥ 8
c) au moins une enzyme exogène susceptible de provoquer l'estérification.

3. Composition cosmétique selon la revendication 1, comprenant :
a) au moins un alcool de formule R₁-OH où R₁ est un groupement alkyle de C₃ à C₃₂, linéaire ou ramifié, saturé ou non-saturé, polyoxyéthyléné ou non, éventuellement substitué.
b) au moins un triglycéride
c) au moins une enzyme exogène susceptible de provoquer l'estérification.

4. Composition cosmétique selon l'une quelconque des revendications 1 à 3, **caractérisée par le fait que** les enzymes sont choisies parmi les lipases, estérases, protéases.

5. Composition cosmétique selon l'une quelconque des revendications 1 à 4, **caractérisée par le fait que** les enzymes peuvent se présenter sous forme libre ou immobilisées sur un support.

6. Composition cosmétique selon l'une quelconque des revendications 1 à 5, **caractérisée par le fait que** les enzymes sont immobilisées sur un support.

7. Composition cosmétique selon l'une quelconque des revendications 1 à 6, **caractérisée par le fait que** la quantité d'enzymes est comprise entre 0,0001 et 50 % en poids par rapport au mélange en poids d'alcools et d'acides ou d'alcools et de triglycérides.

8. Composition cosmétique selon l'une quelconque des revendications 1 à 6, **caractérisée par le fait que** la quantité d'enzymes est comprise entre 0,1 et 20 % en poids par rapport au mélange en poids d'alcools et d'acides ou d'alcools et de triglycérides.

9. Composition cosmétique selon l'une quelconque des revendications 1 à 2 et 4 à 8, **caractérisée par le fait que** la proportion d'acides peut varier entre 1 et 99 % par rapport au mélange en poids d'acides et d'alcools.

10. Composition cosmétique selon l'une quelconque des revendications 1 à 2 et 4 à 8, **caractérisée par le fait que** la proportion d'acides peut varier entre 30 et 70 % par rapport au mélange en poids d'acides et d'alcools.

11. Composition cosmétique selon l'une quelconque des revendications 1 et 3 à 8, **caractérisée par le fait que** la proportion de triglycérides peut varier entre 1 et 99 % par rapport au mélange en poids d'acides et d'alcools.

12. Composition cosmétique selon l'une quelconque des revendications 1 et 3 à 8, **caractérisée par le fait que** la proportion de triglycérides peut varier entre 30 et 70 % par rapport au mélange en poids d'acides et d'alcools.

13. Composition cosmétique selon l'une quelconque des revendications 1 à 12, **caractérisée par le fait que** la proportion d'alcools peut varier entre 1 et 99 % par rapport au mélange en poids d'acides et d'alcools.

14. Composition cosmétique selon l'une quelconque des revendications 1 à 12, **caractérisée par le fait que** la proportion d'alcools peut varier entre 30 et 70 % par rapport au mélange en poids d'acides et d'alcools.

15. Composition cosmétique selon l'une quelconque des revendications 1 à 14, comprenant des adjuvants choisis parmi les huiles carbonées, siliconées, fluorées, les gélifiants, les épaississants, les émollients, les adoucissants, les antioxydants, les opacifiants, les stabilisants, les polymères ioniques, non-ioniques ou amphotères, les vitamines, les parfums, les conservateurs, les colorants, les pigments ou tout autre adjuvant habituellement utilisé en cosmétique, compatibles avec le ou les enzymes utilisés.

16. Composition cosmétique selon l'une quelconque des revendications 1 à 15, **caractérisée par le fait qu'**elle se présente sous la forme de gel, crème ou de lotion.

17. Dispositif à plusieurs compartiments **caractérisé par le fait qu'**il comporte au moins deux compartiments contenant chacun un ou deux des composants suivants:
- un ou des alcools de formule R1-OH où R₁ est un groupement alkyle de C₃ à C₃₂, linéaire ou ramifié, saturé ou non-saturé, polyoxyéthyléné ou non, éventuellement substitué.
- un ou des acides de formule générale R₂-COOH où R₂ est un groupement alkyle de C₃ à C₃₁, linéaire ou ramifié, saturé ou non-saturé, éventuellement substitué avec le nombre de carbones de (R₁ + R₁) ≥ 8 ou un ou des triglycérides
- une ou des enzymes exogènes susceptibles de provoquer l'estérification, l'ensemble du dispositif renfermant au moins un alcool, un acide ou un triglycéride, et une enzyme.

18. Utilisation d'une composition selon l'une quelconque des revendications 1 à 17 pour apporter à la fibre capillaire un aspect doux et brillant.

19. Procédé de traitement cosmétique des cheveux pour apporter un aspect brillant et doux à la fibre capillaire consistant à mélanger sur les cheveux les éléments du dispositif selon la revendication 17 et à appliquer immédiatement la composition résultante sur les fibres kératiniques, à laisser agir pendant 1 à 60 minutes à une température comprise entre 20°C et 65°C et à procéder éventuellement au rinçage à l'eau.

20. Procédé de conditionnement cosmétique des cheveux selon la revendication 19 dans lequel la température est comprise entre 30°C et 65°C.

21. Procédé selon l'une des revendications 19 ou 20, **caractérisé par le fait que** le traitement permettant d'apporter l'aspect brillant et doux comprend une réaction enzymatique *in situ.*
